# EUROPEAN PATENT APPLICATION

(11) **EP 3 736 019 A1**
(43) Date of publication of application: **11.11.2020**
(21) Application number: 20173666.7
(22) Date of filing: 08.05.2020
(51) Int. Cl.: A61N 5/06, A61M 1/00

(54) **WOUND CARE DEVICE**

(30) Priority: 08.05.2019 FI 20195379
(71) Applicant: Led Future Oy, 70210 Kuopio (FI)
(72) Inventor: Hirvonen, Ilkka, 70210 Kuopio (FI); Laitinen, Eero, 70210 Kuopio (FI)
(74) Representative: Kolster Oy Ab

(57) **Abstract**

A wound care device which comprises a UVC-LED radiation source adapted to generate UVC radiation on a target being treated from a bottom surface of the wound care device, control electronics to control the UVC-LED radiation source, a reflection cover, which comprises a trough-shaped structure which passes UVC radiation therethrough, positioned on the bottom surface of the wound care device so that an open edge of the trough-shaped structure is adapted to be placed on the surface of the target being treated.

## Description

### Field of the invention

The invention relates to wound care devices and in particular to disinfecting wound care devices to treat ulcers or similar on a person's or animal's skin.

### Background of the invention

A conventional way to treat a wound, cut, bruise, or similar on the skin of a person or an animal is to clean and disinfect the skin area in question with substances suitable for this purpose. Wounds and bruises are also regularly sprayed with water to accelerate healing and to avoid infections.

However, in many cases a wound does not heal up as desired and may develop an infection, which further adds to the time the wound takes to heal up.

To treat wounds, devices operating by negative pressure, in which an airtight foil is placed over a wound. Between the foil and wound, negative pressure is formed, under the effect of which tissue fluid is sucked from the wound. Such a device is specifically used to treat infected and wide wounds and bruises. The device is used in a long-term manner at a time to be able to accomplish the desired effect.

### Brief description of the invention

It is an object of the invention to develop a wound care device so that the aforementioned problem may be solved. The object of the invention is achieved with a device which is characterised by what is disclosed is the independent claim. Preferred embodiments of the invention are disclosed in the dependent claims.

The invention is based on using a UVC-LED radiation source to generate UVC radiation in such a manner that the tissue of a wound or similar is disinfected. If need be, the device destroys all the microbes in the area being treated by breaking the DNA and RNA chains of cells. UVC radiation additionally destroys pathogens resistant to antibiotics. By adjusting the wavelengths, power and exposure time of the light of the device, it is possible to determine which effects are desired on the target area. The effects are either those that destroy harmful microbes or those that strengthen living tissue. With the inventive device, the closing-up or healing-up time of a wound, bruise or similar may be significantly reduced by means of a quick and simple method of treatment.

According to a preferred embodiment of the invention, the care device additionally comprises a suction feature to form negative pressure on the target being treated, as well as a rinsing feature to rinse the target being treated during treatment. Negative pressure may cause the fluids of the area being treated to come off the wound. Rinsing, for its part, may bring forth the purification from potential impurities in the area being treated.

### Brief description of the drawings

The invention will now be described in more detail in connection with preferred embodiments and with reference to the accompanying drawings, in which:
Figure 1 is a side view of the device according to an embodiment of the invention; and
Figure 2 is an end view of the device of the embodiment of Figure 1.

### Detailed description of the invention

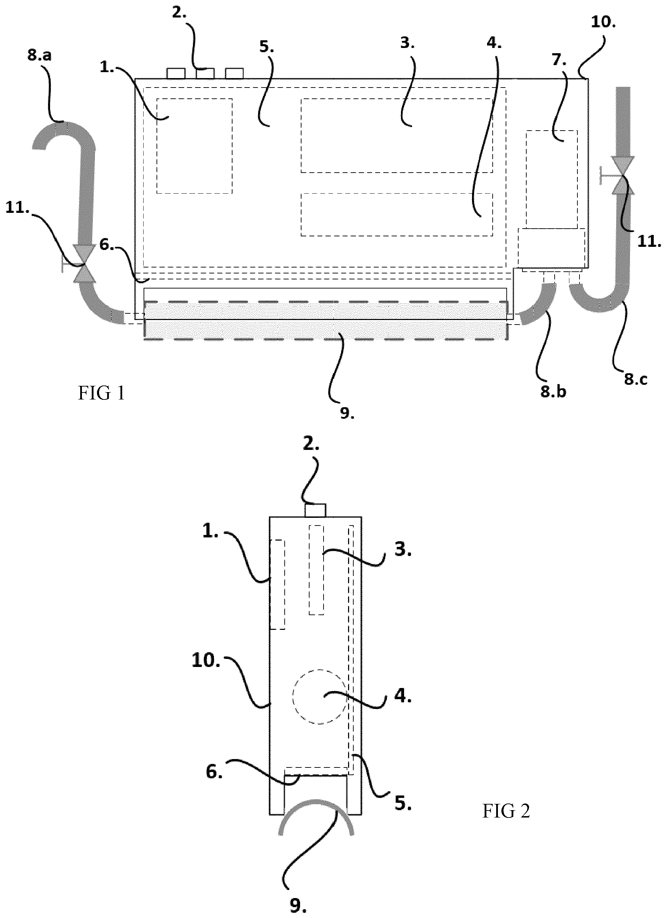

Figure 1 shows an embodiment of the wound care device according to the invention. The wound care device comprises a UVC-LED radiation source. Such a radiation source is typically formed of a plurality of UVC-LED components placed on a circuit board 6. The circuit board 6 is a an elongated circuit board in which the UVC-LED components are located to generate UVC radiation downwards as seen in Figure 1, that is, towards a reflection cover 9.

To generate UVC radiation and to control the radiation source, the device additionally comprises control electronics. The purpose of the control electronics is to produce the desired electric current for the UVC-LED components and to control the operation of the device as is appropriate. To generate electric current, the device additionally comprises a rechargeable battery or a similar source of energy. The intensity of the radiation may be controlled in the prior art manner by controlling the magnitude of the current passing through the UVC-LED components.

The inventive device further has a reflection cover, which comprises a trough-shaped structure that passes UVC radiation therethrough, positioned on the bottom surface of the wound care device so that an open edge of the trough-shaped structure is adapted to be placed on the surface of the target being treated. In Figure 1, the trough-shaped structure 9 is shown as a dotted-line structure. In Figure 2, the numbering thereof corresponding to that of Figure 1, the trough-shaped structure is shown as a curved shape. Figures 1 and 2 make it clear how the circuit board 6 is located on the bottom surface of the device and how the trough-shaped structure is placed on the bottom surface in question. The radiation source is placed on the circuit board, so the UVC radiation is produced from the top side of the trough-shaped structure, as seen in the Figures, in other words the curved outer surface of the trough-shaped structure and the surface 10 of the casing of the device embed the UVC-LED radiation sources. In Figure 2, the cross section of the reflection cover is presented as a semicircle. However, it is obvious that the trough-shaped structure may comprise straight surfaces.

The inventive device allows UVC radiation to be generated on a restricted area of a person's or animal's skin to treat wounds or similar. The trough-shaped structure of the device is pressed onto the area being treated, and the device is caused to generate UVC radiation. Advantageously, there are ready-made radiation programs programmed in the control electronics of the device, which may comprise a treatment time, radiation intensity, radiation intensity variation in pulses, and automatic shut-off at the end of the treatment time. According to an embodiment of the invention, the device has a plurality of UVC-LED components which generate UVC radiation at two wavelengths, at least. The device may thus have a plurality of different UVC-LED components, whereby the wavelength of the radiation generated may be changed according to the need at any one time.

The device is primarily intended for the treatment of wounds, ulcers, and bruises of animals and people. Depending on the use, the wavelength generated by the device varies between 200 and 285 nm. The device also allows the use of UVB light (285 to 315 nm) and UVA light (315 to 400 nm).

According to the preferred embodiment of the invention, the wound care device additionally comprises means for circulating fluid through the trough-shaped structure. The means for circulating fluid advantageously comprise a pump in which there is arranged an inlet for a fluid, means for leading the fluid from the pump to the trough-shaped structure from a first end thereof, and means for removing the fluid from the trough-shaped structure from a second end thereof. The means for leading a fluid from the pump to the trough-shaped structure and for removing it from the through-shaped structure are typically hoses or pipes, which are denoted with numbers 8a, 8b, and 8c in Figure 1. The hose 8c, in particular, is an inlet hose to the pump 7 of the device, which is advantageously a reciprocating pump. The hose 8a is an intermediate hose interconnecting the outlet of the pump and the trough-shaped structure, and the hose 8a is an outlet hose.

A source of fluid may be connected to the inlet hose 8c, such as an infusion bag from which fluid is pumped inside the trough through the first end of the trough. When being used, the trough is pressed on the target being treated whereby the trough and the target being treated form a substantially closed space. The fluid that was pumped into the trough is led out of the trough from its second end.

According to an embodiment of the invention, there are means arranged in the outlet hose to create negative pressure. Negative pressure may be established with a sucking pump, for example. Negative pressure creates a suction from the trough-shaped structure through the outlet hose. The means for forming negative pressure may be adjustable to control the magnitude of negative pressure.

The negative-pressurized trough-shaped structure may be used to suck fluids coming off the target being treated to the trough-shaped structure, and out therefrom through the outlet hose.

The device according to the invention may be used as follows, for example: An infusion bag is connected to the device through the hose 8c, and suction is formed through the hose 8a. First, the medical suction sucks negative pressure through the hose 8a in the area of a wound, while the trough-shaped structure of the device is placed over the target being treated. Negative pressure sucks the fluids on the surface layers of the wound area, together with any microbes, to the wound where they are destroyed by means of UVC radiation. Following the negative-pressurized start, the fluid that has come off the wound area is rinsed off the wound by infusion fluid. The fluid flows along the hose 8c to a reciprocating pump 7, which pumps the fluid through the hose 8b to the trough-shaped structure, formed of quartz glass, for example. Light from UVC LEDs 6 over the trough-shaped structure affects the wound under the trough through the quartz glass and fluid in the trough. The fluid and any material it has released exit the trough along the outlet hose 8a. The pressure in the trough may be adjusted by adjusting a valve in the outlet hose 8a or by means of an adjustable sucking pump.

The pump pumping fluid to the trough-shaped structure may be controlled in a pulse-like manner whereby the rinsing of the wound area may be intensified during the pulses. Operation may be cyclical also so that, following the rinsing periods, negative pressure is re-established in the through-shaped structure to suck and irradiate the fluids. In the inventive device, UVC radiation is generally speaking produced through the trough-shaped structure either so that the trough-shaped structure is void of fluid or so that the trough-shaped structure contains water whereby irradiation takes place through both the trough-shaped structure and a layer of fluid.

To enable fluid rinsing and/or negative pressure within the trough-shaped structure, the ends of the trough-shaped structure must be closed. In other words, there are walls formed in the first and second ends, which extend so as to be flush with the bent walls of the trough-shaped structure. The first and second end have openings formed in them to fasten hoses or similar and to carry fluid to the trough-shaped structure, to produce negative pressure, and to remove fluid from the trough-shaped structure.

The frequency, disinfection time, power and pulsing used by the device may be adjusted with buttons 2 which are operationally connected to the control electronics. The set values are displayed on a screen 1 of the device. The device acquires its power from a battery 4 which is recharged through a charging circuit board 3. The UVC LEDs are controlled by using the control circuit board 5, including the control electronics, according to the values displayed on the screen. All technology is fixed to a device enclosure 10.

The control electronics may include programmable circuits that allow the implementation of different kinds of treatment programs or sequences. The control electronics may be adapted to use a pump by means of which fluid is pumped through the trough-shaped structure. The control electronics may be adapted to use a suction-producing apparatus, such as a pump. Further, the control electronics may be adapted to use valves placed in the inlet hose and/or outlet hose to adjust the flows or pressures.

Those skilled in the art will find it obvious that, as technology advances, the basic idea of the invention may be implemented in many different ways. The invention and its embodiments are thus not restricted to the above-described examples but may vary within the scope of the claims.

## Claims

1. A wound care device, **characterized in that** the wound care device comprises
a UVC-LED radiation source adapted to generate UVC radiation on a target being treated from a bottom surface of the wound care device,
control electronics to control the UVC-LED radiation source,
a reflection cover, which comprises a trough-shaped structure which passes UVC radiation therethrough, positioned on the bottom surface of the wound care device so that an open edge of the trough-shaped structure is adapted to be placed on the surface of the target being treated.

2. A wound care device as claimed in claim 1, **characterized in that** the wound care device comprises means to circulate fluid through the trough-shaped structure.

3. A wound care device as claimed in claim 2, **characterized in that** the means to circulate fluid through the trough-shaped structure comprise a pump having an inlet for a fluid,
means for leading the fluid from the pump to the trough-shaped structure from a first end thereof, and means for removing the fluid from the trough-shaped structure from a second end thereof.

4. A wound care device as claimed in any one of the preceding claims 1 to 3, **characterized in that** the wound care device comprises means to produce negative pressure in the trough-shaped structure.

5. A wound care device as claimed in claim 4, **characterized in that** the means to produce negative pressure comprise a sucking pump adapted in connection with the means adapted to remove a fluid.

6. A wound care device as claimed in claim 4 or 5, **characterized in that** the means to produce negative pressure are adapted to form the means to remove a fluid.

7. A wound care device as claimed in any one of the preceding claims 1 to 6, **characterized in that** the trough-shaped structure comprises end members which extend so as to be flush with the edges of the trough-shaped structure.

8. A wound care device as claimed in any one of the preceding claims 1 to 7, **characterized in that** the trough-shaped structure is formed of quartz glass.

9. A wound care device as claimed in any one of the preceding claims 1 to 8, **characterized in that** the control electronics are adapted to control the circulation of a fluid.

10. A wound care device as claimed in any one of the preceding claims 1 to 9, **characterized in that** the control electronics are adapted to control the UVC-LED radiation source according to a predetermined program.
